Europäisches Patentamt

(19) European Patent Office

Office européen des brevets

(11) Publication number : **0 349 103 B1**

(12) **EUROPEAN PATENT SPECIFICATION**

(45) Date of publication of patent specification :
25.11.92 Bulletin 92/48

(51) Int. Cl.⁵ : **A61K 9/20**

(21) Application number : **89304248.1**

(22) Date of filing : **27.04.89**

(54) **Chewable tablet.**

(30) Priority : **04.05.88 EP 88304008**
**26.08.88 GB 8820265**

(43) Date of publication of application :
**03.01.90 Bulletin 90/01**

(45) Publication of the grant of the patent :
**25.11.92 Bulletin 92/48**

(84) Designated Contracting States :
**AT BE CH DE ES FR GB GR IT LI LU NL SE**

(56) References cited :
**EP-A- 0 003 589**
**EP-A- 0 273 005**
**CA-A- 1 229 552**
**FR-A- 2 175 853**
**FR-A- 2 197 592**

(73) Proprietor : **SMITH KLINE & FRENCH
LABORATORIES LIMITED
Mundells
Welwyn Garden City Hertfordshire, AL7 1EY
(GB)**

(72) Inventor : **France, Gordon**
**SmithKline Beecham, Mundells**
**Welwyn Garden City, Hertfordshire AL71EY**
**(GB)**
Inventor : **Leonard, Graham Stanley**
**SmithKline Beecham, Mundells**
**Welwyn Garden City, Hertfordshire AL71EY**
**(GB)**

(74) Representative : **Hutchins, Michael Richard,
Dr. et al**
**Smith Kline & French Laboratories Limited**
**Corporate Patents Mundells**
**Welwyn Garden City Hertfordshire AL7 1EY**
**(GB)**

## Description

The present invention relates to solid pharmaceutical compositions such as chewable tablets, particularly those containing histamine $H_2$-receptor antagonists such as cimetidine, and to methods for preparing such compositions.

Chewable tablets are often employed when the active ingredient is intended to act in a localised manner, rather than systemically. For example, antacids are often administered in chewable tablet form. Chewable tablets can also be employed as an alternative to administering a number of smaller tablets when the active ingredient requires a relatively large dose in order to achieve the desired therapeutic effect. A further reason for using chewable tablets, as distinct from tablets which are intended to be swallowed intact, is to enable the tablet to be reduced to a finely divided state quickly, thereby facilitating more rapid release and hence more rapid absorption of the active ingredients. Chewable tablets can thus be useful for the treatment of conditions where a quick onset of action of the active ingredient is required. One such condition is gastro-oesophageal reflux disease (GORD) in which quick control of gastric acidity is desirable in order to minimise the adverse effects of acid reflux. Histamine $H_2$-receptor antagonists, such as cimetidine, have been shown, or would be expected, to be useful in the treatment of GORD, and the provision of a chewable tablet containing such $H_2$-antagonists represents one object of the present invention.

It is generally recognised (see, for example, EP 0190826) that patient compliance with a drug treatment regimen can be a problem when the drug has an unpleasant taste or mouth feel and this has prompted numerous investigations into methods of improving palatability.

The provision of palatable dosage forms represents a particular problem when the dosage form is a chewable tablet, i.e. a tablet intended to disintegrate in the mouth under the action of chewing or sucking and where, in consequence, the unpleasant-tasting active ingredient has ample opportunity to come into contact with the bitter-taste receptors on the tongue.

One known approach to the solution of this problem is to coat the drug with a coating agent which prevents it from coming into contact with the taste-buds. Such an approach can have certain drawbacks; firstly the coating agent may be removed by the mechanical grinding action of the teeth during chewing; and secondly the presence of a substantial layer of coating agent can inhibit release of the drug in the gastrointestinal tract and thereby lower its bioavailability.

A further known approach is to adsorb the drug onto a suitable substrate thereby also preventing contact of the drug with the taste-buds. This approach is described in US Patent No. 4,647,459.

European Patent Application 0190826 describes a method for masking the unpleasant taste of a substance by forming the unpleasant-tasting substance into an aggregate along with a pre-swelled substantially anhydrous hydrocolloid. The hydrocolloid absorbs saliva and acquires a slippery texture which enables it to lubricate the particles of aggregate and mask the unpleasant gritty texture of drugs such as cholestyramine, and dietary fibre supplements such as locust bean gum. The preparation of the aggregates involves preparing an aqueous composition of the hydrocolloid, contacting the aqueous composition with the unpleasantly textured substance to form the aggregates, followed by drying the aggregates. The preferred and exemplified method of preparing such an aggregate involves the use of a fluidised bed granulator.

The above-mentioned methods of masking the unpleasant tastes and textures of certain medicaments involve incorporating the medicament into the aggregate or granule.

It has now been found that the unpleasant tastes of certain medicaments, for example the intensely bitter taste of histamine $H_2$-receptor antagonists such as cimetidine, can be reduced or eliminated by employing a water-insoluble hygroscopic excipient such as microcrystalline cellulose in a particular amount as an extragranular excipient.

The use of extragranular microcrystalline cellulose in a solid dosage form is described in EP 0196546. However, the particular use described in EP 0196546 is use as a cushioning agent between coated granules to prevent fracturing of the granule-coating during a compression step. No mention is made of any taste-masking properties nor is it suggested that such dosage forms could be in the form of chewable tablets. The use of extragranular explotab™ as a disintegrant in swallow tablets is described in CA-A- 1229 552.

In a first aspect, the present invention provides a pharmaceutical chewable tablet comprising:
(i) granules containing a histamine $H_2$-receptor antagonist; and
(ii) an extragranular water-insoluble hygroscopic excipient in an amount of 5% to 15% by weight of the total weight of the tablet.

Chewable tablets are characterised in that they are typically larger than tablets which are intended to be swallowed; for example typically the total weight of such a tablet is at least 1g and typically the minimum distance across the centre of the largest face of the tablet is at least 10mm, e.g. in the range 10-20mm, and is suitably at least 15mm. For example such a tablet can have a square cross-section wherein the sides of the square are at least 10mm in length or it can be circular in cross-section such that the diameter of the circle is at least 10mm. Alternatively, or additionally, chewable tablets typically are characterised in that they contain flavouring and/or sweetening agents. In the present context, the term sweetening agents is intended to mean sweeteners other than sweet-tasting sugars, sugar alcohols and oligo- and polysaccharides, although such substances can also be included in the tablets. Thus, for example it is intended to refer to sweeteners such as ammonium glycyrrhizinate, and artificial sweeteners such as sodium cyclamate, sodium saccharinate and aspartame.

Flavouring agents can be natural in origin or synthetically obtained and can be employed to impart a variety of different tastes to the tablet, for example butterscotch, aniseed, mint and various fruit flavours or combinations thereof. Such flavouring agents are well known in the art of pharmacy and need not be described in detail here.

Chewable tablets are generally uncoated, i.e. they do not usually have a surface coating of a release-retarding or controlling substance.

The term insoluble as used herein refers to the definition in the U.S. Pharmacopoeia National Formulary USP XXI, 1985, whereby 10,000 or more parts of solvent are required to dissolve 1 part of solute.

Suitably the hygroscopic substance has the ability to absorb at least 5% by weight (relative to its own weight) of water in an atmosphere of 90% humidity. More usually it will have the ability to absorb about 10% or more, by weight, of water.

The water-insoluble hygroscopic excipient is suitably an organic substance and is preferably polymeric in nature; for example it can be a polysaccharide. Typically the hygroscopic excipient is chosen from the group of substances comprising underivatised celluloses such as powdered celluloses and microcrystalline celluloses; derivatised celluloses such as cross-linked carboxymethylcelluloses, e.g. the sodium and calcium cross-linked carboxymethylcelluloses; sodium starch glycolate and cross-linked polyvinylpyrrolidone.

Powdered cellulose is defined in the U.S. Pharmacopoeia National Formulary USP XXI (1985), page 1547, as being a purified, mechanically disintegrated cellulose prepared by processing alpha cellulose obtained as a pulp from fibrous plant materials. It is described as containing not less than 97.0% and not more than 102.0% of cellulose calculated on the dried basis.

Microcrystalline cellulose is defined in the U.S. Pharmacopoeia National Formulary USP XXI (1985), page 1546, as being partially depolymerised cellulose obtained by treating fibrous plant material-derived alpha cellulose with mineral acids. As with the powdered cellulose, it is described as containing 97.0-102.0% of cellulose calculated on the dried basis.

Particular examples of celluloses are microcrystalline celluloses such as Emcocel™, (supplied by Edward Mendell of New York) and Avicel™ (supplied by FMC Corporation of Philadelphia, PA). Particular grades of Avicel™ include Avicel PH 103, Avicel PH 101 and Avicel PH 105. Further examples of celluloses are powdered celluloses such as Elcema™ (supplied by Degussa of Frankfurt).

Examples of cross-linked carboxymethylcelluloses (croscarmelloses) include the sodium salt Ac-Di-Sol and the calcium salt ECG 505 (both supplied by FMC Corporation).

Examples of sodium starch glycolate include Explotab™ which is supplied by Edward Mendell of New York, see also U.S.P. 3,034,911; and an example of a cross-linked polyvinylpyrrolidone is Kollidon™ CL which is supplied by BASF of the Federal Republic of Germany.

The water-insoluble hygroscopic substance typically constitutes 7-13% (w/w), for example 9% (w/w) of the total weight of the tablet. Usually it is particulate in nature and suitably substantially all of the particles of hygroscopic substance will be less than 300μm in size, and the particle size typically will be in the range 20-150μm, for example 100μm.

The histamine $H_2$-receptor antagonist can be any such substance which is capable of being administered orally but the compositions of the present invention are particularly advantageous when the substance has at least very slight solubility in water, i.e. it is soluble to the extent of at least 1 part in 10,000 parts of water.

Examples of $H_2$-antagonists include cimetidine, ranitidine, famotidine, nizatidine and roxatidine.

The compositions of the present invention are particularly useful for substances which have an unpalatable taste, for example a bitter taste. Such substances include, for example, cimetidine and ranitidine.

The histamine $H_2$-receptor antagonist can be granulated in accordance with standard pharmaceutical techniques; thus it can be mixed with a solution of a binding agent in a conventional mixing device or it can be subjected to fluidised bed granulation methods as known in the art.

In a second aspect, the present invention provides a solid pharmaceutical composition comprising cimetidine-containing granules, and an extragranular water-insoluble hygroscopic excipient in an amount of 5% to 15% (w/w) of the composition.

The composition typically contains 7-13% by weight of the hygroscopic excipient, for example 9%.

The water-insoluble hygroscopic excipients are characterised as described hereinabove and particular examples of such excipients and their physical characteristics, e.g. particle size, are also as described hereinabove.

Preferred excipients for use in combination with cimetidine include particulate underivatised celluloses such as microcrystalline and powdered celluloses, e.g. the Avicels™.

The granules of cimetidine can comprise an additional taste-masking agent. For example, the granules can be formed from a mixture of cimetidine and Eudragit™ E as described in European patent applications numbers 294933 and 290229.

Particular dosage forms in accordance with the present invention are chewable tablets. Such tablets normally contain at least 75 mg of cimetidine. As a maximum the tablet will not normally contain more than 800 mg of cimetidine. Preferably it contains 100 or 200 mg of cimetidine.

The chewable tablets of the present invention can also contain solid diluents such as sugars and sugar alcohols, for example lactose, xylitol, sorbitol and mannitol. Where desired additional sweeteners can be added, for example ammonium glycyrrhizinate, sodium cyclamate, sodium saccharinate and aspartame as well as flavours and additional taste maskers, for example sodium chloride and Contramarum.

The tablets can also contain other standard tableting excipients; for example a disintegrant. It will be appreciated that when the disintegrant is a cross-linked carboxymethylcellulose, sodium starch glycolate or cross-linked polyvinylpyrrolidone, it can also function as an extragranular, hygroscopic, water-insoluble excipient as defined hereinabove.

In one particular embodiment of the invention, there is provided a chewable tablet comprising granules containing a total of 200 mg of cimetidine, the amount of cimetidine corresponding to 12.5% by weight of the tablet; 70% w/w lactose and/or sorbitol, and, as hygroscopic water-insoluble excipients, 2.5% w/w croscarmellose sodium and 9.5% microcrystalline cellulose.

The cimetidine compositions of the invention can also contain a hydroxide or carbonate antacid. Examples of suitable antacids include aluminium hydroxide, magnesium hydroxide, magnesium carbonate, calcium carbonate and co-dried gels for example aluminium hydroxide-magnesium carbonate co-dried gel. In practice the quantity of antacid is between 5 milli-equivalents per tablet and 30 milli-equivalents, typically 14 milli-equivalents.

Where the tablet contains an antacid, preferably the antacid is pre-compressed or granulated before it is mixed with the cimetidine granules, for example as described in European Patent Application Number 0 294 933 and as described in Example 1 of this application.

The granules can be sieved to remove fine particles and larger particles. Preferably the granules pass through a 1.4 mm sieve but are retained by a 0.2 mm sieve.

The antacid can be pre-compressed or granulated by standard methods.

The following Examples illustrate the invention.

EXAMPLE 1

## 200 mg Cimetidine/Antacid Chewable Tablet

### Ingredient

| Cimetidine Premix Granules | mg/tablet | %w/w |
|---|---|---|
| Cimetidine | 200.0 | 90.9 |
| Eudragit™ E100 | 20.0 | 9.1 |

### Antacid (Al/Mg) Granules

|  | mg/tablet | %w/w |
|---|---|---|
| Sorbitol: Direct Compression Grade | 590.0 | 34.01 |
| Lactose: Direct Compression Grade | | |
| Spray dried | 325.0 | 18.73 |
| Crystalline | 325.0 | 18.73 |
| Dried Aluminium Hydroxide Gel | 250.0 | 14.41 |
| Magnesium Hydroxide | 200.0 | 11.53 |
| Croscarmellose Sodium Type A[+] | 30.0 | 1.73 |
| Magnesium Stearate | 15.0 | 0.86 |
| | 1735.0 | 100.00 |

### Tableting Mix for Compression

|  | mg/tablet |
|---|---|
| Cimetidine Premix Granules | 220.0 |
| Antacid (Al/Mg) Granules | 1735.0 |
| Microcrystalline Cellulose (Avicel™ PH102)[+] | 200.0 |
| Aspartame | 10.0 |
| Aniseed | 20.0 |
| Butterscotch | 20.0 |
| Magnesium Stearate | 15.0 |
| TOTAL | 2220.0 |

[+] Croscarmellose Sodium Type A and Avicel™ PH102 can both be obtained from the FMC Corporation, Philadelphia PA.

### Process Description

A 40% w/v solution of the Eudragit™ E100 in methylene chloride was added with mixing to the cimetidine and blended until granules were formed. The resulting granules were dried and then sieved through a 1.0mm (16 mesh) screen.

The aluminium hydroxide, magnesium hydroxide and other ingredients for the antacid granules were sieved through a 1.4 mm (12 mesh) screen and mixed together. The resulting mix was compressed on a rotary tablet press and the resulting compacts were milled using a 12 mesh screen.

The cimetidine granules, antacid granules and extragranular excipients were put into a cone blender and mixed thoroughly. The resulting mix was discharged from the blender and compressed on a suitable rotary tablet press fitted with the appropriate punches.

## EXAMPLE 2

### 200 mg Cimetidine Chewable Tablet

#### Ingredient

| | mg/tablet | %w/w |
|---|---|---|
| Cimetidine | 200.0 | 12.7 |
| Eudragit™ E100 | 20.0 | 1.3 |
| Sorbitol: Direct Compression Grade | 600.0 | 38.0 |
| Lactose: Direct Compression Grade | 500.0 | 31.6 |
| Croscarmellose Sodium Type A | 40.0 | 2.5 |
| Aspartame | 10.0 | 0.6 |
| Aniseed Flavouring | 20.0 | 1.3 |
| Butterscotch Flavouring | 20.0 | 1.3 |
| Magnesium Stearate | 20.0 | 1.3 |
| Microcrystalline Cellulose (Avicel™ PH102) | 150.0 | 9.5 |
| | 1580.0 | |

The cimetidine and Eudragit™ E100 were granulated in the manner described in Example 1 and the resulting granules were compressed together with the remaining ingredients to form tablets.

## Claims

**Claims for the following Contracting States : AT, BE, CH, DE, FR, GB, IT, LI, LU, NL, SE**

1. A pharmaceutical chewable tablet composition comprising:
   (i) granules containing a histamine $H_2$-receptor antagonist; and
   (ii) an extragranular water-insoluble hygroscopic excipient in an amount of 5% to 15% by weight of the total weight of the tablet.

2. A composition according to claim 1 wherein the amount of hygroscopic excipient is in the range 7-13% by weight of the tablet.

3. A composition according to claim 2 wherein the amount of excipient is 9% by weight of the tablet.

4. A composition according to any one of claims 1 to 3 wherein the hygroscopic excipient is chosen from the group of substances comprising underivatised celluloses, cross-linked carboxymethylcelluloses, sodium starch glycolate and cross-linked polyvinylpyrrolidone.

5. A solid pharmaceutical composition comprising cimetidine-containing granules, and an extragranular water-insoluble hygroscopic excipient in an amount of 5% to 15% (w/w) of the composition.

6. A composition according to claim 5 wherein the hygroscopic excipient is microcrystalline cellulose or powdered cellulose.

7. A composition according to claim 6 wherein the cellulose comprises particles, substantially none of which have a size greater than $300\mu m$.

8. A composition according to any one of claims 5 to 7 which contains an antacid.

9. A pharmaceutical chewable tablet composition comprising granules containing a total of 200 mg of cimetidine, the amount of cimetidine corresponding to 12.5% by weight of the tablet; 70% w/w lactose and/or sorbitol, and, as hygroscopic water-insoluble excipients, 2.5% w/w croscarmellose sodium and 9.5% microcrystalline cellulose.

**Claims for the following Contracting States : ES, GR**

1. A process for preparing a pharmaceutical chewable tablet composition comprising:
   (i) granules containing a histamine $H_2$-receptor antagonist; and
   (ii) an extragranular water-insoluble hygroscopic excipient in an amount of 5% to 15% by weight of the total weight of the tablet;
   which process comprises forming the histamine $H_2$-receptor antagonist into granules, mixing the granules with the hygroscopic excipient and optionally accessory ingredients and excipients, and compressing the resulting mixture to form a chewable tablet.

2. A process according to claim 1 wherein the amount of hygroscopic excipient is in the range 7-13% by weight of the tablet.

3. A process according to claim 2 wherein the amount of excipient is 9% by weight of the tablet.

4. A process according to any one of claims 1 to 3 wherein the hygroscopic excipient is chosen from the group of substances comprising underivatised celluloses, cross-linked carboxymethylcelluloses, sodium starch glycolate and cross-linked polyvinylpyrrolidone.

5. A process for preparing a solid pharmaceutical composition comprising cimetidine-containing granules, and an extragranular water-insoluble hygroscopic excipient in an amount of 5% to 15% (w/w) of the composition, which process comprises forming the cimetidine into granules, bringing the granules into association with the hygroscopic excipient and optionally accessory ingredients and excipients, to form the solid composition.

6. A process according to claim 5 wherein the hygroscopic excipient is microcrystalline cellulose or powdered cellulose.

7. A process according to claim 6 wherein the cellulose comprises particles, substantially none of which have a size greater than $300\mu m$.

8. A process according to any one of claims 5 to 7 wherein the composition contains an antacid.

9. A process for preparing a pharmaceutical chewable tablet composition comprising granules containing a total of 200 mg of cimetidine, the amount of cimetidine corresponding to 12.5% by weight of the tablet; 70% w/w lactose and/or sorbitol, and, as hygroscopic water-insoluble excipients, 2.5% w/w croscarmellose sodium and 9.5% microcrystalline cellulose, which process comprises forming the cimetidine into granules, mixing the granules with the lactose and/or sorbitol and the hygroscopic water-insoluble excipients and compressing the resulting mixture to form a chewable tablet.

**Patentansprüche**

**Patentansprüche für folgende Vertragsstaaten : AT, BE, CH, DE, FR, GB, IT, LI, LU, NL, SE**

1. Arzneimitttel in Form einer Kautablette, umfassend:
   (i) ein Granulat, das einen Histamin $H_2$-Rezeptor-Antagonisten enthält; und
   (ii) einen extragranularen, wasserunlöslichen, hygroskopischen Arzneistoffträger in einer Menge von 5 bis 15 Gew.-% des Gesamtgewichts der Tablette.

2. Mittel gemäß Anspruch 1, wobei die Menge des hygroskopischen Arzneistoffträgers im Bereich von 7 bis 13 Gew.-% der Tablette liegt.

3. Mittel gemäß Anspruch 2, wobei die Menge des Arzneistoffträgers 9 Gew.-% der Tablette ist.

4. Mittel gemäß einem der Ansprüche 1 bis 3, wobei der hygroskopische Arzneistoffträger aus der Substanzgruppe, umfassend nichtderivatisierte Cellulosen, vernetzte Carboxymethylcellulosen, Natriumstärkeglycolat und vernetztes Polyvinylpyrrolidon, ausgewählt ist.

5. Festes Arzneimittel, umfassend ein Cimetidin enthaltendes Granulat, und einen extragranularen, wasserunlöslichen, hygroskopischen Arzneistoffträger in einer Menge von 5 bis 15 Gew.-% des Arzneimittels.

6. Mittel gemäß Anspruch 5, wobei der hygroskopische Arzneistoffträger mikrokristalline Cellulose oder auf Staubfeinheit gemahlene Cellulose ist.

7. Mittel gemäß Anspruch 6, wobei die Cellulose Teilchen umfaßt, von denen praktisch keines eine Größe über 300 µm aufweist.

8. Mittel gemäß einem der Ansprüche 5 bis 7, das ein Antazidum enthält.

9. Arzneimittel in Form einer Kautablette, umfassend ein Granulat, das eine Gesamtmenge von 200 mg Cimetidin, wobei die Menge Cimetidin 12,5 Gew.-% der Tablette entspricht; 70 Gew.-% Lactose und/oder Sorbitol, und als hygroskopische, wasserunlösliche Arzneistoffträger 2,5 Gew.-% des Natriumsalzes der vernetzten Carboxymethylcellulose und 9,5 Gew.-% mikrokristalline Cellulose enthält.

**Patentansprüche für folgende Vertragsstaaten : ES, GR**

1. Verfahren zur Herstellung eines Arzneimitttel in Form einer Kautablette, umfassend:
   (i) ein Granulat, das einen Histamin $H_2$-Rezeptor-Antagonisten enthält; und
   (ii) einen extragranularen, wasserunlöslichen, hygroskopischen Arzneistoffträger in einer Menge von 5 bis 15 Gew.-% des Gesamtgewichts der Tablette;
   wobei das Verfahren das Formen des Histamin $H_2$-Rezeptor-antagonisten zu Körnern, das Mischen der Körner mit dem hygroskopischen Arzneistoffträger und gegebenenfalls begleitenden Inhaltsstoffen und Arzneistoffträgern und das Zusammenpressen des resultierenden Gemischs zur Erzeugung einer Kautablette umfaßt.

2. Verfahren gemäß Anspruch 1, wobei die Menge des hygroskopischen Arzneistoffträgers im Bereich von 7 bis 13 Gew.-% der Tablette liegt.

3. Verfahren gemäß Anspruch 2, wobei die Menge des Arzneistoffträgers 9 Gew.-% der Tablette ist.

4. Verfahren gemäß einem der Ansprüche 1 bis 3, wobei der hygroskopische Arzneistoffträger aus der Substanzgruppe, umfassend nichtderivatisierte Cellulosen, vernetzte Carboxymethylcellulosen, Natriumstärkeglycolat und vernetztes Polyvinylpyrrolidon, ausgewählt ist.

5. Verfahren zur Herstellung eines festen Arzneimittels, umfassend ein Cimetidin enthaltendes Granulat, und einen extragranularen, wasserunlöslichen, hygroskopischen Arzneistoffträger in einer Menge von 5 bis 15 Gew.-% des Arzneimittels; wobei das Verfahren das Formen von Cimetidin zu Körnern, die Vereinigung der Körner mit dem hygroskopischen Arzneistoffträger und gegebenenfalls begleitenden Inhaltsstoffen und Arzneistoffträgern zur Erzeugung des festen Mittels umfaßt.

**6.** Verfahren gemäß Anspruch 5, wobei der hygroskopische Arzneistoffträger mikrokristalline Cellulose oder auf Staubfeinheit gemahlene Cellulose ist.

**7.** Verfahren gemäß Anspruch 6, wobei die Cellulose Teilchen umfaßt, von denen praktisch keines eine Größe über 300 µm aufweist.

**8.** Verfahren gemäß einem der Ansprüche 5 bis 7, wobei das Mittel ein Antazidum enthält.

**9.** Verfahren zur Herstellung eines Arzneimittels in Form einer Kautablette, umfassend ein Granulat, das eine Gesamtmenge von 200 mg Cimetidin, wobei die Menge Cimetidin 12,5 Gew.-% der Tablette entspricht; 70 Gew.-% Lactose und/oder Sorbitol, und als hygroskopische, wasserunlösliche Arzneistoffträger 2,5 Gew.-% des Natriumsalzes der vernetzten Carboxymethylcellulose und 9,5 Gew.-% mikrokristalline Cellulose enthält; wobei das Verfahren das Formen von Cimetidin zu Körnern, das Mischen der Körner mit Lactose und/oder Sorbitol und das Zusammenpressen des resultierenden Gemischs zur Erzeugung einer Kautablette umfaßt.


**Revendications**

**Revendications pour les Etats contractants suivants : AT, BE, CH, DE, FR, GB, IT, LI, LU, NL, SE**

**1.** Composition pharmaceutique de tablette à mâcher comprenant :
(i) des granules contenant un antagoniste de récepteur $H_2$ histaminique ; et
(ii) un excipient hygroscopique insoluble dans l'eau extra-granulaire en une quantité de 5 % à 15 % en poids du poids total de la tablette.

**2.** Composition suivant la revendication 1, dans laquelle la quantité d'excipient hygroscopique est de l'ordre de 7 à 13 % en poids de la tablette.

**3.** Composition suivant la revendication 2, dans laquelle la quantité d'excipient est de 9 % en poids de la tablette.

**4.** Composition suivant l'une quelconque des revendications 1 à 3, dans laquelle l'excipient hygroscopique est choisi dans le groupe de substances comprenant des celluloses non dérivées, des carboxyméthylcelluloses réticulées, un amidon glycolate de sodium et une polyvinyl-pyrrolidone réticulée.

**5.** Composition pharmaceutique solide comprenant des granules contenant de la cimétidine et un excipient hygroscopique insoluble dans l'eau extra-granulaire en une quantité de 5 % à 15 % en p/p de la composition.

**6.** Composition suivant la revendication 5, dans laquelle l'excipient hygroscopique est une cellulose microcristalline ou une cellulose en poudre.

**7.** Composition suivant la revendication 6, dans laquelle la cellulose comprend des particules dont pratiquement aucune n'a une dimension supérieure à 300 µm.

**8.** Composition suivant l'une quelconque des revendications 5 à 7, qui contient un anti-acide.

**9.** Composition pharmaceutique de tablette à mâcher comprenant des granules contenant un total de 200 mg de cimétidine, la quantité de cimétidine correspondant à 12,5 % en poids de la tablette ; 70 % en p/p de lactose et/ou de sorbitol et, en tant qu'excipients hygroscopiques insolubles dans l'eau, 2,5 % en p/p de croscarmellose de sodium et 9,5 % de cellulose micro-cristalline.

**Revendications pour les Etats contractants suivants : ES, GR**

**1.** Procédé pour préparer une composition pharmaceutique de tablette à mâcher comprenant :
(i) des granules contenant un antagoniste de récepteur $H_2$ histaminique ; et
(ii) un excipient hygroscopique insoluble dans l'eau extra-granulaire en une quantité de 5 % à 15 % en poids du poids total de la tablette ;
lequel procédé comprend la mise de l'antagoniste de récepteur $H_2$ histaminique sous forme de granules, le mélange des granules avec l'excipient hygroscopique et éventuellement d'autres composants et excipients, et la compression du mélange résultant pour former une tablette à mâcher.

2. Procédé suivant la revendication 1, dans lequel la quantité d'excipient hygroscopique est de l'ordre de 7 à 13 % en poids de la tablette.

3. Procédé suivant la revendication 2, dans lequel la quantité d'excipient est de 9 % en poids de la tablette.

4. Procédé suivant l'une quelconque des revendications 1 à 3, dans lequel l'excipient hygroscopique est choisi dans le groupe de substances comprenant des celluloses non-dérivées, des carboxyméthylcelluloses réticulées, un amidon glycolate de sodium et une polyvinyl-pyrrolidone réticulée.

5. Procédé pour préparer une composition pharmaceutique solide comprenant des granules comprenant de la cimétidine et un excipient hygroscopique insoluble extra-granulaire en une quantité de 5 % à 15 % en p/p de la composition, lequel procédé comprend la mise de la cimétidine sous forme de granules, l'association des granules avec l'excipient hygroscopique et éventuellement d'autres composants et excipients pour former la composition solide.

6. Procédé suivant la revendication 5, dans lequel l'excipient hygroscopique est une cellulose microcristalline ou une cellulose en poudre.

7. Procédé suivant la revendication 6, dans lequel la cellulose comprend des particules dont pratiquement aucune n'a une dimension supérieure à 300 $\mu$m.

8. Procédé suivant l'une quelconque des revendications 5 à 7, dans lequel la composition contient un antiacide.

9. Procédé pour préparer une composition pharmaceutique de tablette à mâcher comprenant des granules contenant un total de 200 mg de cimétidine, la quantité de cimétidine correspondant à 12,5 % en poids de la tablette ; 70 % en p/p de lactose et/ou de sorbitol et, en tant qu'excipients hygroscopiques insolubles dans l'eau, 2,5 % en p/p de croscarmellose de sodium et 9,5 % de cellulose microcristalline, lequel procédé comprend la mise de la cimétidine sous forme de granules, le mélange des granules avec le lactose et/ou le sorbitol et les excipients hygroscopiques insolubles dans l'eau et la compression du mélange résultant pour former une tablette à mâcher.